(19)

Europäisches
Patentamt
European
Patent Office
Office européen
des brevets

(11) **EP 4 546 203 A1**

(12) **EUROPEAN PATENT APPLICATION**

(43) Date of publication:
**30.04.2025  Bulletin 2025/18**

(21) Application number: **24208442.4**

(22) Date of filing: **23.10.2024**

(51) International Patent Classification (IPC):
**G06F 30/23** (2020.01)       **H01M 10/0525** (2010.01)
**G06F 111/10** (2020.01)        **G06F 119/04** (2020.01)
**H01M 4/02** (2006.01)

(52) Cooperative Patent Classification (CPC):
**H01M 10/0525; G06F 30/23; G06F 30/28;**
G06F 2111/10; G06F 2119/04; G06F 2119/08;
H01M 2004/021

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **23.10.2023   US 202363592325 P**

(71) Applicant: **Dassault Systemes Americas Corp.
Waltham, MA 02451 (US)**

(72) Inventors:
• **CARLSON, Timothy
  Hayward (US)**

• **Salazar-Tio, Rafael
  San Ramon (US)**
• **HAHN, YOUNGWON
  Everett (US)**
• **OANCEA, Victor
  East Greenwich (US)**
• **Balasubramanian, Ganapathi Raman
  Pleasanton (US)**
• **CROUSE, Bernd
  Berkeley (US)**
• **ISLAM, Ashraful
  Daly City (US)**

(74) Representative: **Bandpay & Greuter
11 rue Christophe Colomb
75008 Paris (FR)**

(54) **METHOD UPSCALING FROM MICROSTRUCTURE TO CONTINUUM USING A MESOSCALE, HETEROGENEOUS HOMOGENIZATION**

(57)     A microstructure is upscaled to generate a coarsened heterogeneous spatial distribution of porosity and a set of porosity dependent constitutive relationships. A three dimensional (3D) microstructure model, bulk material properties, and/or porosity is received for anode, cathode, and separator battery components. A coarsened porosity model with emergent properties is calculated from the battery component microstructures as a function of the porosity. Bruggeman coefficients for each battery component sub region are calculated from the effective ionic conductivity, electric and thermal conductivity, and ionic diffusivity. A heterogeneous mesoscale 3D battery model is created by combining the anode, cathode, and separator materials into a single cell structure and separately partitioning each into coarse voxels to create a 3D model of porosity.

FIG. 1

EP 4 546 203 A1

**Description**

CROSS-REFERENCE TO RELATED APPLICATIONS

This application claims the benefit of U.S. Provisional Patent Application serial number 63/592,325, filed October 23, 2023, entitled "Method for electrochemistry modeling of Li-ion Battery by upscaling from microstructure to continuum using a mesoscale, heterogeneous homogenization," which is incorporated by reference herein in its entirety.

FIELD OF THE INVENTION

The present invention relates to sustainable energy storage using Li-ion batteries and related electrochemistry simulation, and more particularly, is related to a framework for determining heterogeneity effects on battery performance.

BACKGROUND OF THE INVENTION

In scientific literature there are two types of electrochemical models for Li-ion batteries, both with shortcomings relating to capturing performance and aging drivers in a practical way:

(1) Continuum modeling, like finite element (FE) analysis, where elements are in the millimeter range with degrees of freedom accounting for homogenized material properties (chemical composition plus modeled microstructure behavior). While this provides the ability to simulate a full cell battery, continuum modeling either uses generic empirical relationships for modeled microstructure behavior, or requires laboratory measurements which may be difficult to obtain, and

(2) Microstructure modeling, where sub-micrometer resolution is able to capture the real microstructure behavior, but only on small samples sizes. Microstructure modeling provides accurate microstructure behavior instead of generic empirical relationships and less need of difficult to obtain laboratory data. However, it is not feasible to simulate the behavior of a full cell with microstructure modeling due to the intractable number of degrees of freedom required.

The accuracy resulting from property heterogeneity captured by microstructure modeling is important for performance, aging, and degradation simulation, but the required model sizes for a full cell level simulation are impractical to archive with current computing capability. As such, currently there is no complete solution based upon the microstructure modeling approach. Existing continuum models typically assign uniform material properties to each material (anode, cathode, and separator), which (incorrectly) assumes that any existing heterogeneities can be neglected. Therefore, there is a need to upscale the heterogeneities from microstructure models into a continuum model.

SUMMARY OF THE INVENTION

Embodiments of the present invention provide a method to connect a fine resolution battery microstructure characterization (micrometer scale) and a coarse resolution battery electrochemical simulation (millimeter scale), while maintaining the fine resolution heterogeneity effects on performance, aging, and degradation, all within a reasonable, workable coarse resolution model size.

A microstructure is upscaled to generate a coarsened heterogeneous spatial distribution of porosity and a set of porosity dependent constitutive relationships. A three dimensional (3D) microstructure model, bulk material properties, and/or porosity is received for anode, cathode, and separator battery components. A coarsened porosity model with emergent properties is calculated from the battery component microstructures as a function of the porosity. Bruggeman coefficients for each battery component sub region are calculated from the effective ionic conductivity, electric and thermal conductivity, and ionic diffusivity. A heterogeneous mesoscale 3D battery model is created by combining the anode, cathode, and separator materials into a single cell structure and separately partitioning each into coarse voxels to create a 3D model of porosity.

Other systems, methods and features of the present invention will be or become apparent to one having ordinary skill in the art upon examining the following drawings and detailed description. It is intended that all such additional systems, methods, and features be included in this description, be within the scope of the present invention and protected by the accompanying claims.

In examples, the method of the invention may comprise one or more of the following:

- the porosity of the received bulk material properties comprises a fine resolution model comprising a plurality of elements, wherein each element is characterized as either a solid having a porosity of substantially zero or a pore having porosity of 100%;

- calculating emergent properties from the battery component microstructures as a function of the porosity further comprises the steps of:

  computing an average porosity in sub regions of each material;
  assigning each element of the coarsened porosity model the average porosity for all elements within a region; and
  assigning the average porosities to individual elements of a finite element mesh;

- the voxels are uniquely defined with a uniform voxel size throughout for each of the anode, cathode, and separator;
- creating an Abaqus input file using the coarsened image and constitutive relationships;
- receiving the coarsened porosity model and associated constitutive relationships are received as input into a continuum electrochemical solver;
- determining effective properties as a function of saturation;
- computing a capillary pressure vs saturation curve; and calculating a change in pore pressure with saturation changes;
- simulating a 3D Newman model (165), wherein the continuum electrochemical solver comprises a 3D Newman model simulator;
- computing performance and aging metrics (170) from degrees of freedom of the 3D Newman model (165)
- determining a point where lithium plating on a surface of the 3D Newman model (165) becomes thermodynamically favorable;

- optimizing microstructure of the anode and cathode materials of the 3D Newman model;
- introducing a plurality of high porosity channels into anode and cathode materials of the 3D Newman model;
- printing the heterogeneous mesoscale 3D battery model to a file; and/or
- providing the heterogeneous mesoscale 3D battery model file to a continuum modeler.

BRIEF DESCRIPTION OF THE DRAWINGS

The accompanying drawings are included to provide a further understanding of the invention, and are incorporated in and constitute a part of this specification. The components in the drawings are not necessarily to scale, emphasis instead being placed upon clearly illustrating the principles of the present invention. The drawings illustrate embodiments of the invention and, together with the description, serve to explain the principles of the invention.

FIG. 1 is a flowchart of an overview of an exemplary first embodiment of a workflow a simulation framework.
FIG. 2 illustrates two contrasting examples of tortuosity.
FIG. 3 illustrates an exemplary process of fitting Bruggeman coefficients/constitutive relationships.
FIG. 4 is a sequence of four plots illustrating examples of computable constitutive relationships.
FIG. 5 introduces base and homogeneous cases for material properties.
FIG. 6 shows voltage/OCV vs SOC curves at 3C (top) and 6C (bottom) comparing base and homogeneous cells.
FIG. 7 illustrates an example of a coarsening process to obtain Heterogeneous and Homogeneous battery cells.
FIG. 8 is a detail of FIG. 7 illustrating the relative porosity of the cathode, separator, and anode.
FIG. 9 shows plots illustrating Voltage/OCV vs SOC curves at 3C (top) and 6C (bottom) comparing homogeneous and heterogeneous cells.
FIG. 10 shows plots of anode plating potential (EPOTLPL) at face of the separator vs SOC at 3C and 6C.
FIG. 11 shows a distribution of plating potential vs porosity (top), a graphic illustration of where plating occurs (center), and a distribution of plating potential in heterogeneous and homogenous cells (bottom).
FIG. 12 shows contour plots of plating potential (EPOTLPL) at face of anode near separator at the end of a charge phase, comparing homogeneous and heterogeneous cells.
FIG. 13 is an illustration of heterogeneous and optimized cell porosity distributions.
FIG. 14 shows plots of Voltage/OCV vs SOC curves at 2.4C (top) and 4.8C (bottom) comparing heterogeneous and optimized cells.
FIG. 15 shows plots of anode plating potential (EPOTLPL) at face of separator vs SOC at 2.4C and 4.8C comparing heterogeneous and optimized cells.
FIG. 16 shows a distribution of EPOTLPL vs Porosity (top) and distribution of EPOTLPL in heterogeneous and optimized cells (bottom).
FIG. 17 shows contour plots of EPOTLPL at face of anode near separator at the end of a charge phase, comparing heterogeneous and optimized cells.
FIG. 18 is a flowchart of an exemplary embodiment of a method for upscaling a microstructure to a continuum.
FIG. 19 is a schematic diagram illustrating an example of a system for executing functionality of the present invention.

DETAILED DESCRIPTION

The following definitions are useful for interpreting terms applied to features of the embodiments disclosed herein, and are meant only to define elements within the disclosure.

As used within this disclosure, "Abaqus" refers to a software suite for finite element analysis and computer-aided engineering, originally released in 1978. Abaqus is a suite of engineering analysis software packages used to simulate the physical response of structures and solid bodies to various environmental conditions. Abaqus is used by engineers to simulate complex problems in a variety of industries. As used within this disclosure, the "Newman Model" refers to a theoretical electrochemistry framework utilized by Abaqus to simulate the charging and discharging of battery cells. Coupled with thermal, swelling, and elasticity theory, Abaqus provides a framework to simulate battery Multiphysics to aid in the design and optimization of battery cells. The Newman Model may be used, for example, to calculate concentrations, potentials, temperature, displacements, and pore pressure as degrees of freedom.

As used within this disclosure, a "Bruggeman relationship" and "Bruggeman exponent" refers to a relationship between porosity and tortuosity for granular porous media. The Bruggeman relationship is an inverse power law of porosity to an exponent, called the Bruggeman exponent, which is typically assumed to have a value of 0.5.

As used within this disclosure, "continuum modeling" (Finite Element (FE) analysis) refers to a method for numerically solving the partial differential equations associated with the degrees of freedom (concentrations, electric potentials, et cetera) defined in the continuum that would be unfeasible to solve analytically. A continuum model is a representation of a process or structure that gradually changes between two defined points. These points may be tangible, for example, numerical or temporal, or intangible. The exemplary embodiments herein refer to the Abaqus software suite for continuum modeling.

As used within this disclosure, "microstructure modeling" refers to computing transport properties, among others, considering the explicit presence of a porous media at the scale in which individual pores/solid phases are distinguishable and separated, and effective transport properties can be obtained, adding the effect of the microstructure to the pure material transport properties. The exemplary embodiments herein use DigitalROCK software, for digital pore-scale simulation of microstructure material properties, providing, for example, pore space analysis, MICP curves, absolute permeability, relative permeability, capillary desaturation, tortuosity, ionic conductivity and diffusivity, and electric and thermal conductivities.

As used within this disclosure, a "battery" or "battery cell" refers to an energy storage device, for example to a Li-ion battery, among others.

As used within this disclosure, "C rate" refers to a multiple of the current required to charge a battery in 1 hour.

As used within this disclosure, "tortuosity" refers to a measure of the shortest path through a material in a given direction.

As used within this disclosure, a "grid" or a "computational grid" generally refers to a geometrically defined region of a battery cell, typically at a layer and/or the layers, such as anode, cathode, and separator, used for simulation in lieu of the entire layered structure.

Reference will now be made in detail to embodiments of the present invention, examples of which are illustrated in the accompanying drawings. Wherever possible, the same reference numbers are used in the drawings and the description to refer to the same or like parts.

Design and optimization of battery cells is an expensive, time consuming, and difficult process that typically relies on laboratory testing of the different components, materials, and processes that leads to the final battery cell. A process that replicates these components, including cell design as well as materials microstructures and chemical composition, can speed up the battery optimization process, according to required metrics such as performance, aging, and degradation as well as related quantities such as energy density or charging speed.

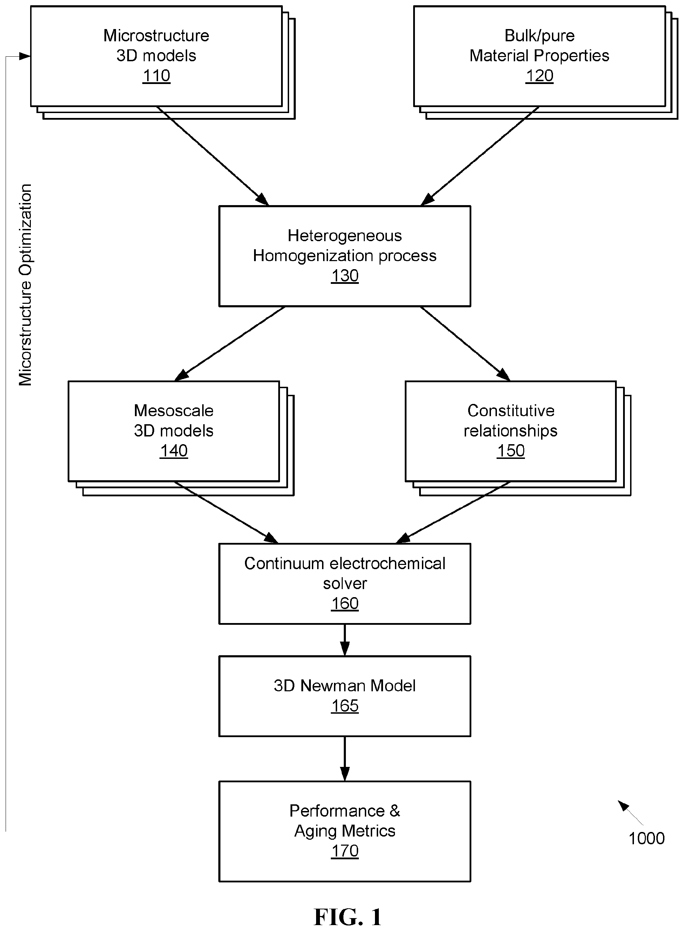

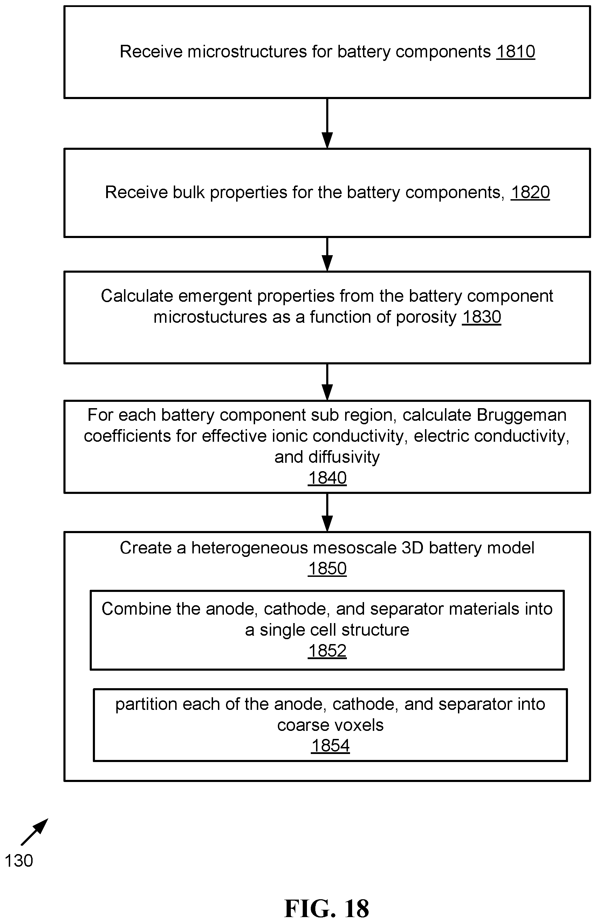

The exemplary embodiment herein provide an efficient way to effectively upscale a battery microstructure (provided by DigitalROCK) to a full continuum model (provided to Abaqus), enabling an efficient modeling of a full Li-ion battery cell, accounting for heterogeneities without introducing an unfeasible number of degrees of freedom. The exemplary embodiments combine the benefits of microstructure characterization and continuum modeling while minimizing their respective individual disadvantages. As shown by FIG. 1, the embodiments take a representative 3D microstructure model 110 for each electrode/separator in a battery cell and the corresponding material properties 120 as inputs to a heterogeneous homogenization process 130. For example, the representative 3D microstructure models 110 and the corresponding material properties 120 may be provided by DigitalROCK. The heterogeneous homogenization process 130 generates two outcomes: a coarsened heterogeneous spatial distribution of porosity (mesoscale 3D coarsened porosity models 140) and a set of porosity dependent constitutive relationships 150 for other properties (for example, ionic diffusivity and conductivity, electric and thermal conductivity, saturation vs pressure, specific surface, and linear elastic coefficients). The heterogeneous homogenization process 130 is described in further detail below with reference to FIG. 18.

The coarsened porosity model 140 and associated constitutive relationships 150 are received as input into a continuum electrochemical solver 160, such as Abaqus, which includes a 3D Newman model simulator to produce a 3D Newman model 165, where performance and aging metrics 170 may be computed from degrees of freedom of the model 165. A full microstructure optimization cycle may be realized by repeating this sequence of steps while changing the electrode/separator microstructures. This full workflow takes the heterogeneity required for performance and aging into account within a feasible computational model size. The embodiments provide a simulation framework 1000 that accounts for property heterogeneities in a full battery cell simulation, that fully capture the correct local and global behavior of these metrics, in particular performance and aging prediction.

The simulations of these embodiments may be incorporated into the manufacture of battery cells, for example, providing parameter inputs directly into battery manufacturing equipment, such as particle size distribution, amount of additives, like carbon binder domain, and target porosity for the electrodes by controlling the calendaring applied pressure. Further, the simulations represent the behavior of real electrodes with structured heterogeneity and provide geometric inputs during related manufacturing processes, for example, the spacing of ablations or micro channels.

The exemplary embodiments provide a mesoscale model of a battery cell that maintains heterogeneities without introducing an unfeasible number of degrees of freedom. The embodiments homogenize sub-regions of the anode, cathode, and separator to introduce a heterogeneous porosity/saturation distribution without completely abandoning the assumptions inherent to the industry standard Newman model (that is, that it can be assumed that there is particle and electrolyte at all material points). To homogenize material properties, effective properties are calculated as a function of parameters related to characteristics of the specific microstructure, such as porosity and saturation. The embodiments use these constitutive relationships as inputs in Abaqus electrochemistry simulations, wherein the battery cells analyzed have a heterogeneous porosity distribution determined from the average porosity of sub volumes of the scanned materials. With this workflow, the distribution, and therefore the material microstructure, may be adjusted or optimized to meet performance goals or limit the negative effects of aging. The details of this process are presented below, along with some representative results.

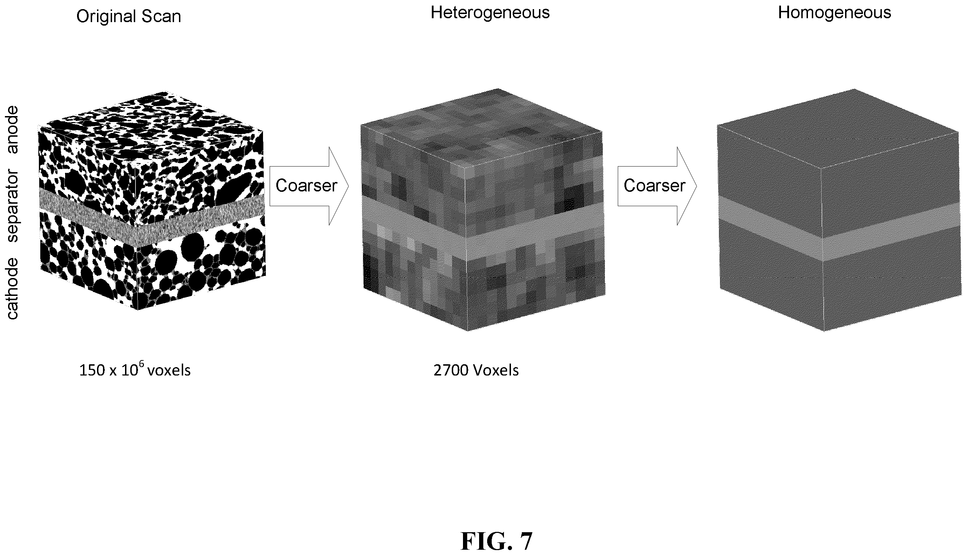

The exemplary embodiments start by receiving fully resolved microstructure 3D models 110 and material properties 120 for the anode, cathode, and separator components in order to establish an accurate mesoscale for continuum modeling in Abaqus. These 3D models may be obtained, for example, directly from real materials via a FIB-SEM (focused ion beam scanning electron microscopy) or x-ray micro-tomography 3D imaging. Alternatively, these materials can be procedurally generated via simulation of the calendaring process on assortments of particles in Abaqus followed by a procedural introduction of additives such as those present in the Carbon Binder Domain (CBD), or via a custom battery microstructure generation tool such as MATBOX from NREL (see Allen, J.M., Chang, J., Usseglio-Viretta, F.L.E. et al. "A Segregated Approach for Modeling the Electrochemistry in the 3-D Microstructure of Li-Ion Batteries and Its Acceleration Using Block Preconditioners". J Sci Comput 86, 42 (2021)). See FIG. 7 (left) for an example of a fully resolved microstructure.

Additionally, given that all material constitutive relationships 150 at least implicitly depend on the bulk properties 120 of the materials used, the exemplary embodiments receive bulk material properties for ionic conductivity, diffusivity, electric conductivity, among others. These properties may be obtained via the literature (see Ecker et. al, 2014 e.g., Madeleine Ecker et. al. "Parameterization of a Physico-Chemical Model of a Lithium-Ion Battery: I. Determination of Parameters" J. Electrochem. Soc. 162 A1836 (2015), and Madeleine Ecker et. al. "Parameterization of a Physico-Chemical Model of a Lithium-Ion Battery: II. Model Validation" J. Electrochem. Soc. 162 A1849 (2015)) or molecular modeling and simulation, using Materials Studio, for example.

The exemplary embodiments create mesoscale 3D models 140 by coarsening the original 3D microstructural material models 110 based on porosity, which varies spatially. In the original fine resolution model, each element is mostly solid (porosity zero) or pore (porosity 100%), while in the new coarsened porosity model each element has an average porosity of all the small elements within the same region. The method accomplishes this by computing the average porosity in sub regions of each material and then systematically assigning these porosities to individual elements of a finite element mesh, for example, via a script. For example, each element might correspond to the average porosity in the corresponding 50 pixel cube region of the original scan, if the original image is to be broken up into cubes with 50 pixel side lengths.

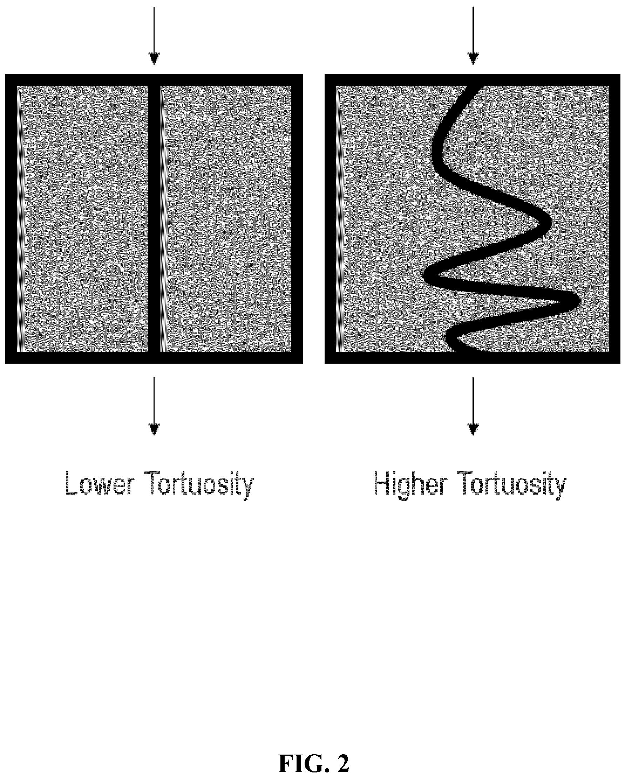

For porous/nonhomogeneous media, most effective properties that arise from the material microstructure homogenization can be computed from bulk material properties and the material porosity (or, alternatively, solid volume fraction). Given that these effective properties are used in the Newman model, effective properties adequately reflect the material microstructure in order to obtain results that accurately reflect those materials at hand. An example of effective material properties that can be computed via porosity are the effective ionic conductivity and effective diffusivity of the electrolyte, which are calculated in a similar manner as a function of porosity, tortuosity, and bulk electrolyte conductivity/diffusivity. Eq. 2 and Eq. 3 are functional expressions relating effective ionic conductivity (Eq. 2) to bulk ionic conductivity and effective diffusivity (Eq. 3) to bulk ionic diffusivity in orthotropic materials. The tortuosity in a given direction, which is a measure of the shortest path through a material, as illustrated in FIG. 2, may be itself regarded as a function of the porosity through the well-established, although empirical, Bruggeman relationship,

$$\tau_e = \varepsilon_e^{-\alpha} \quad (\text{Eq. 1})$$

where $\tau_e$ is the tortuosity of the electrolyte in a given direction, $\varepsilon_e$ is the porosity, and $\alpha$ is the Bruggeman exponent for the given direction. The Bruggeman exponent is usually assumed to be 0.5, which corresponds to the packing of uniform spheres. However, for real materials this exponent can vary quite substantially from this value.

$$K_{eff} = K_e \epsilon_e \begin{bmatrix} \frac{1}{\tau_{ex}} & 0 & 0 \\ 0 & \frac{1}{\tau_{ey}} & 0 \\ 0 & 0 & \frac{1}{\tau_{ez}} \end{bmatrix} = K_e \begin{bmatrix} \epsilon_e^{\alpha_x+1} & 0 & 0 \\ 0 & \epsilon_e^{\alpha_y+1} & 0 \\ 0 & 0 & \epsilon_e^{\alpha_y+1} \end{bmatrix} \quad (\text{Eq. 2})$$

$$D_{eff} = D_e \epsilon_e \begin{bmatrix} \frac{1}{\tau_{ex}} & 0 & 0 \\ 0 & \frac{1}{\tau_{ey}} & 0 \\ 0 & 0 & \frac{1}{\tau_{ez}} \end{bmatrix} = D_e \begin{bmatrix} \epsilon_e^{\alpha_x+1} & 0 & 0 \\ 0 & \epsilon_e^{\alpha_y+1} & 0 \\ 0 & 0 & \epsilon_e^{\alpha_y+1} \end{bmatrix} \quad (\text{Eq. 3})$$

Eq. 2 provides anisotropic effective ionic conductivity and Eq. 3 provides anisotropic effective diffusivity as a function of porosity via the Bruggeman relationship. Here, $K_e$ is the bulk ionic conductivity and $D_e$ is the bulk diffusivity.

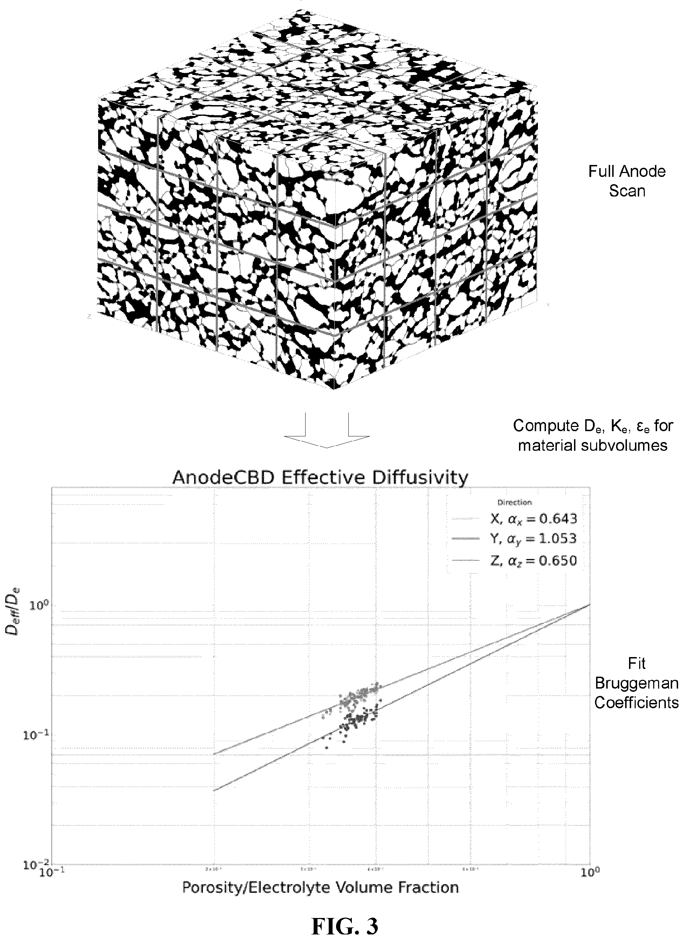

As shown by FIG. 3, To calculate the Bruggeman exponents, the original image of each material is broken down into a number of sub volumes. The method that computes effective diffusivity is then run on each sub-volume, for example, via a simulation code, calculating a single effective diffusivity for each sub volume, along with the porosity in the sub-volume. The effective diffusivity is then plotted against the porosity, and the Bruggeman exponent may then be fit from the bulk diffusivity and the constitutive relationships given by Eqs. 2 and 3.

Given that the Bruggeman coefficient corresponds to the tortuosity of the microstructure, the same exponent can be used to describe the effective ionic conductivity curve. Although the equations describing the relationship between effective properties and porosity do not always follow the Bruggeman relationship (for example, in the case of effective thermal conductivity of the electrode materials) this same strategy is employed to develop porosity dependency curves for all effective quantities.

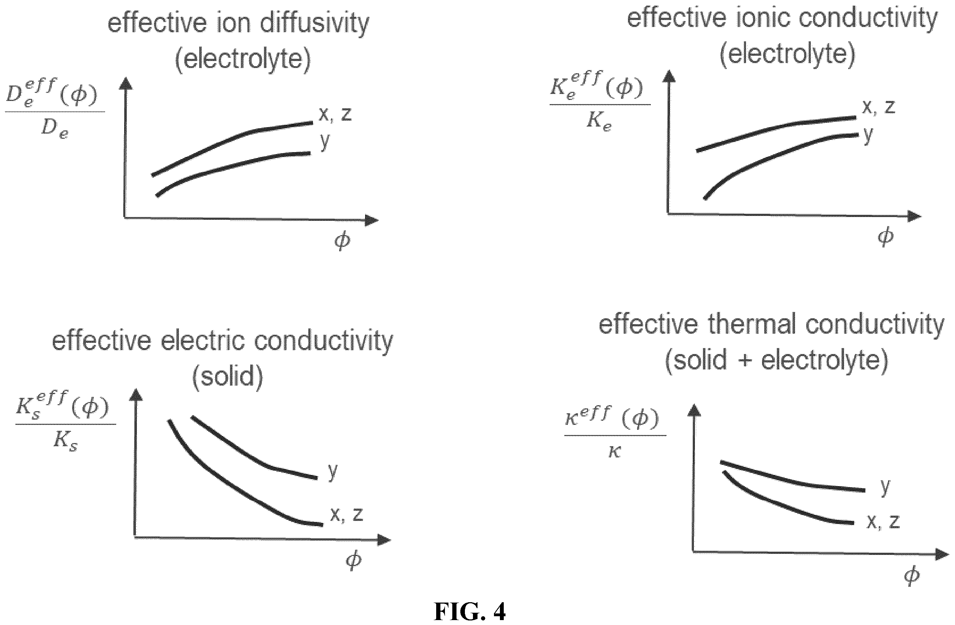

The effective properties may be computed as a function of an additional independent variable: saturation. Changes in saturation affect the amount of electrolyte in the medium, which can impact the connectivity of the electrolyte and therefore the effective properties influenced by the presence of electrolyte. Additionally, capillary pressure vs saturation curves can be computed using a multiphase fluid flow Lattice Boltzmann solver, such as PowerFLOW, which are used to calculate changes in pore pressure with saturation changes in the fully coupled electrochemical-thermal-mechanical-pore pressure simulation in an FE implementation of the 3D Newman model in Abaqus, for instance. While the range of effective material properties described herein as a part of this workflow are shown in FIG. 4, in alternative embodiments the methodology may be extended to other properties and fields, for example, saturation vs pore pressure, porosity vs active surface area, porosity vs. elastic modulus, covariation of all properties with saturation, among others.

A complete set of partial differential equations for the electrochemical, thermal, and mechanical behavior of a battery is implemented in the Newman model 165. The degrees of freedom for the model include the Lithium concertation in the liquid and solid phases of the electrodes and separator, the electric and ionic potentials in the solid and liquid phases respectively, and the temperature. A 3D model with Anode, Cathode, and separator regions 140 is received as input, and traditionally effective properties are computed based on the assumption of uniformly packed spheres. However, it is possible to allow for arbitrary variations on these properties following an input porosity filed, such as the one computed in the heterogeneous homogenization step. Along with the arbitrarily variable porosity field, all other material properties associated with porosity via the constitutive relationship 150 may be used instead of and/or in addition to the traditional relationships.

The embodiments focus on two metrics 170 for performance and aging, computed from the outcomes of the Newman model simulation 165. For performance, the method focuses on the internal resistance of the battery cells, which can be computed as the difference between the voltage and the OCV, all divided by the current. For Aging the method focuses on the plating potential, which is the difference between the solid potential and the electrolyte potential in the region of the Anode near the separator. Lithium plating can only occur when this potential is negative, with more plating being correlated with a lower potential. Lithium plating is a negative aging effect that takes out available mobile Lithium from the charging/discharging process and converts it in immobile solid metallic lithium that slowly grows as crystalline structures called dendrites.

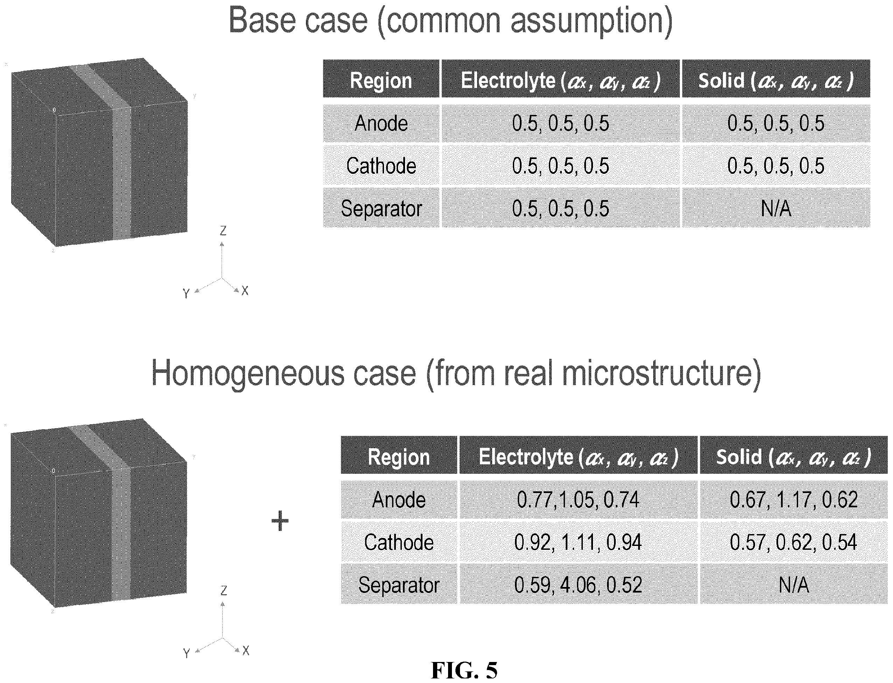

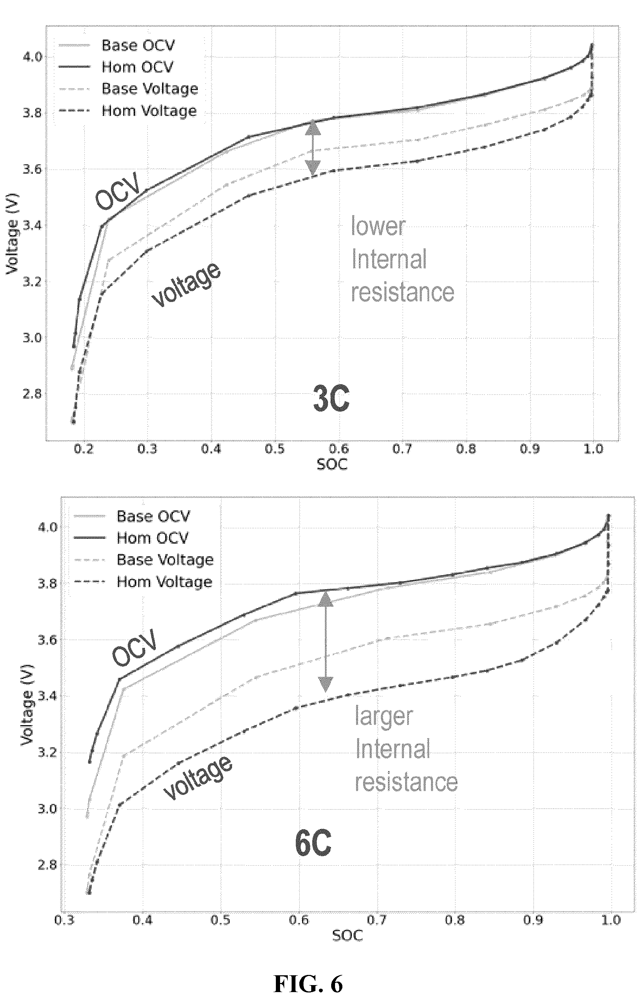

Contrary to the typical assumptions of battery modeling, the Bruggeman exponents for battery materials are not necessarily isotropic, nor are they necessarily close to 0.5, and nor are they the same between the solid and electrolyte. Consider the cells shown in FIG. 5, where the only differences are the Bruggeman coefficients assigned to the solid and electrolyte. Under identical boundary conditions/loading, it is determined that the model that implements the common assumption of 0.5 Bruggeman coefficients for both phases significantly overestimate the performance of the battery microstructure. In particular, the internal resistance of the cell, which can be determined by the difference between the open circuit voltage (OCV) and the measured voltage divided by the current (which is the same constant in both cases), is clearly higher in the representative homogeneous cell as shown in FIG. 6. The difference between the cells is only amplified when the discharge current is doubled (i.e., when the battery is charged at 6C rather than 3C, where 1C is the current that needs to be constantly applied to fully discharge a battery in one hour). At certain points of the 6C discharge simulation, the difference in internal resistance is upwards of 100 percent. See FIG. 6 for plots of OCV/Voltage versus state of charge at 3C and 6C during the discharge phase. From this, it can be concluded that effective properties which actually reflect the microstructure at hand are critical for simulation accuracy. The workflow of the embodiments allows for efficient computation of these material properties, which is not found in any available electrochemical simulation software.

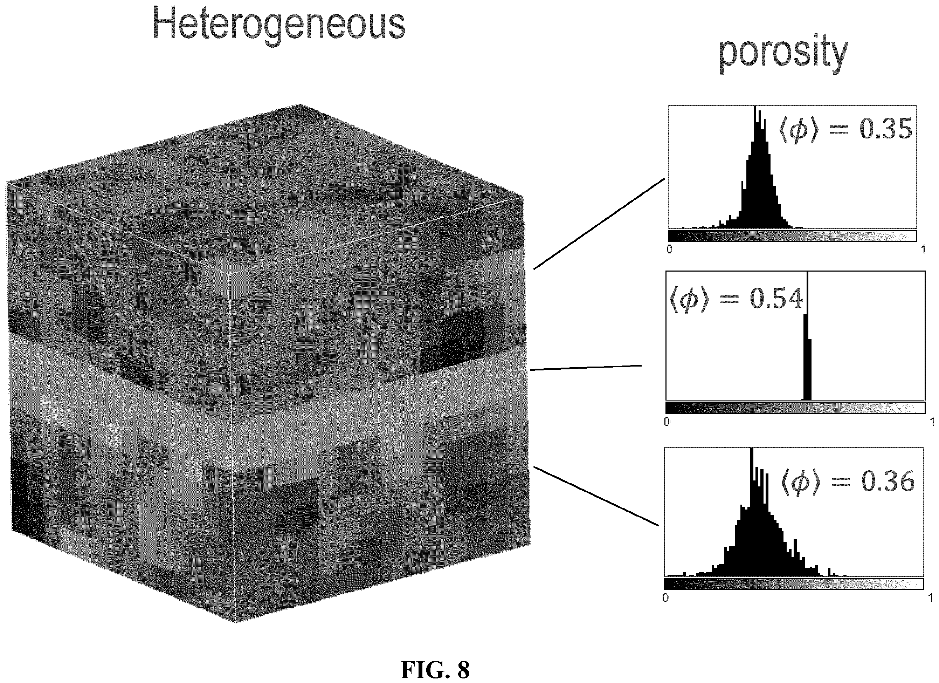

The constitutive relationships calculated are a consequence of the heterogeneous nature of the microstructure of the battery materials considered. However, in typical battery modeling applications, battery materials are assigned a single initial porosity/saturation, which completely neglects the remaining contribution of heterogeneities to the performance and aging of battery cells. The workflow of the embodiments assigns a heterogeneous distribution of porosity and saturation at different levels of coarsening. Since most effective material properties are a function of these variables, this is equivalent to assigning each element in a mesh with a different effective ionic conductivity, diffusivity, et cetera. This allows for the creation of a heterogeneous mesoscale battery cell, where enough homogenization is done so that the Newman model can still be effectively utilized, but the heterogeneous distribution of porosity is maintained. This is not accomplished in the case of full homogenization (see FIGS. 7-8). As such, the mesoscale cell, henceforth referred to as the heterogeneous cell, maintains the heterogeneity of the original scan of the microstructure but with a computational efficiency far closer to the fully homogenized cell.

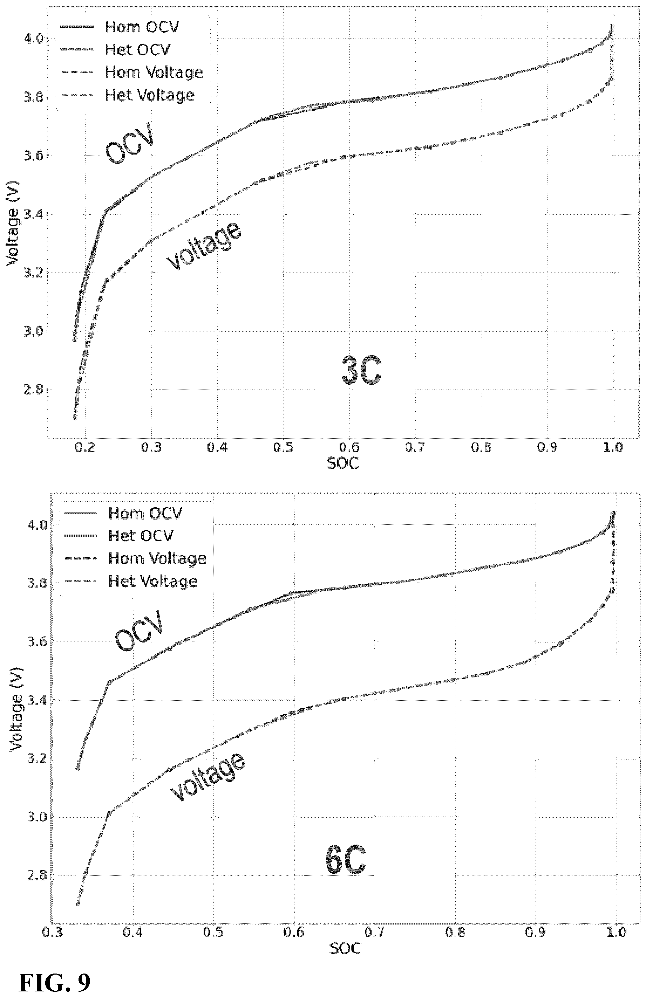

In simulating charge/discharge cycles of the heterogeneous and homogeneous cells, the same effective material properties are used; these constitutive relationships were calculated from material microstructure images via the workflow procedure described above. Globally, there are only subtle differences in performance between the homogeneous and heterogenous cells, as shown in FIG. 9, although it is expected that these differences may be more pronounced at higher C rates and as more material properties are considered as functions of spatially varying variables like porosity. FIG. 9 shows plots of OCV and Voltage versus SOC at 3C and 6C, respectively. The constitutive relationships for thermal conductivity are included in this analysis in addition to the effective properties following the Bruggeman relationships.

A starker difference between the heterogeneous and homogeneous cells becomes apparent when considering the various aging mechanisms batteries are expected to experience. One of these phenomena is known as lithium plating, where metallic lithium may form on the surface of the anode particles, usually in the region bordering the separator. This process is generally assumed to be irreversible, and removes available active surface areas and cyclable lithium from future charge/discharge cycles. Experimental evidence indicates that different regions of the battery will plate to different extents, and simulations also indicate the spatial extent of plating is correlated with porosity (see Andrew Colclasure "Quantifying Heterogeneities/Degradation During Fast Charge" Presentation, DOE Vehicle Technologies Profram 2020 Annual Merit Review and Peer Evaluation Meeting, June 1-4, 2020, and Matthew Keyser, Kandler Smith, Hakim Iddir, et. al. "Understanding Impact of Local Heterogeneities During Fast Charge" Presentation, U.S. Department of Energy Vehicle Technologies Office Annual Merit Review, Arlington VA, June 10-13, 2019, e.g.). Therefore, when the heterogeneities are included in the model, different aging behavior in regions of different local porosity may be captured.

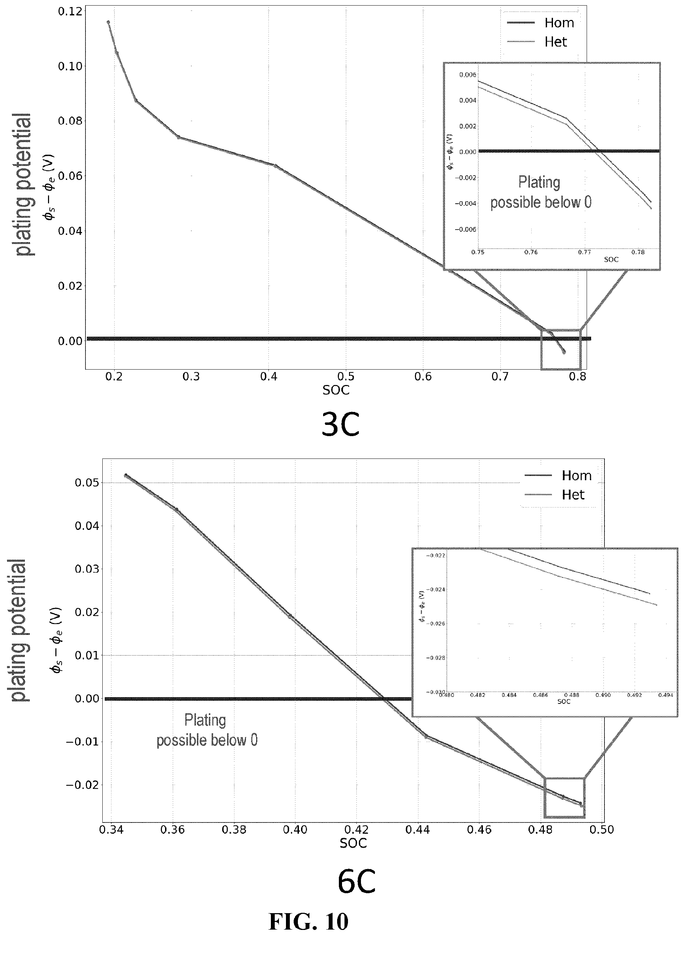

While the actual mechanics of lithium plating are not modeled explicitly, i.e., the growth of lithium on the surface of particles, the point at which lithium plating becomes thermodynamically favorable can be determined. Lithium plating is only possible when the potential difference between the solid and liquid in the anode is negative. FIG. 10 shows a plot of this difference at the integration points closest to the separator vs state of charge in the heterogeneous and homogeneous cells during a charging cycle at 3C and 6C. For the homogeneous cell, only the average difference is reported at each point, as it is essentially constant, but for the heterogeneous case the average potential of all points near the separator is plotted along with the average only in regions of unusually high or low porosity to show the full range of potentials. It is clear that plating occurs at a slightly higher state of charge in the heterogeneous cell than in the homogeneous cell. Accordingly, at each state of charge the potential difference is higher in the heterogeneous cell, even at the extremes.

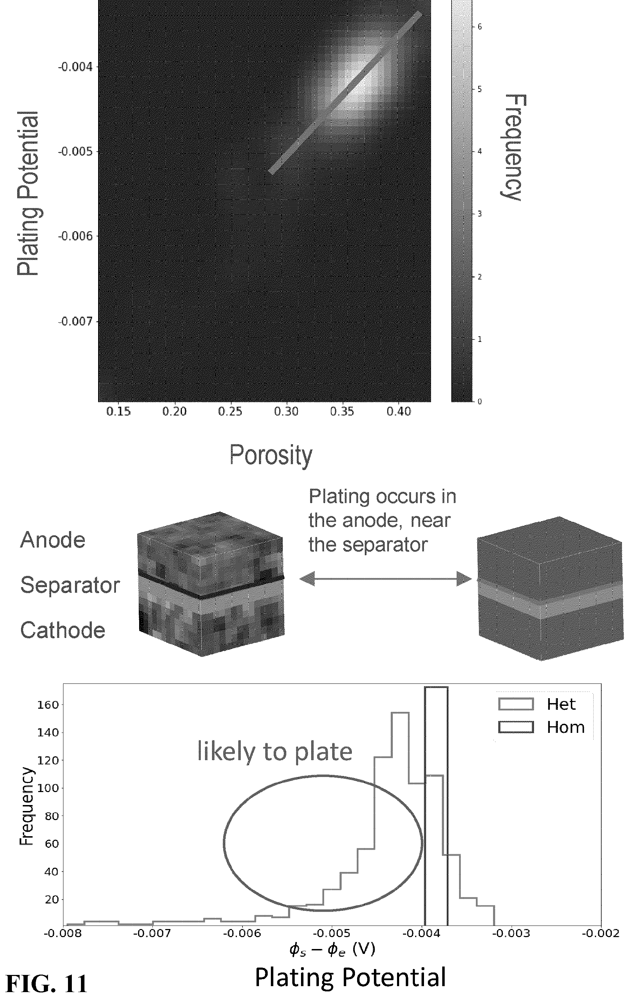

The starkest differences come when one investigates the distribution of the difference in potentials, henceforth referred to as the lithium plating potential (EPOTLPL), and how this distribution correlates with the porosity distribution. It is apparent from the simulations that EPOTLPL is correlated with porosity, as expected. Furthermore, the EPOTLPL has a wide distribution in the heterogeneous cell, whereas the distribution in the homogeneous cell can be simplified to a delta function. FIG. 11 shows these distributions, along with a graphic showing the region of the model where the data comes from. The top graphic illustrates the distribution of plating potential vs porosity, the center shows a graphic illustration of where plating occurs, and the bottom plots the distribution of plating potential in heterogeneous and homogenous cells.

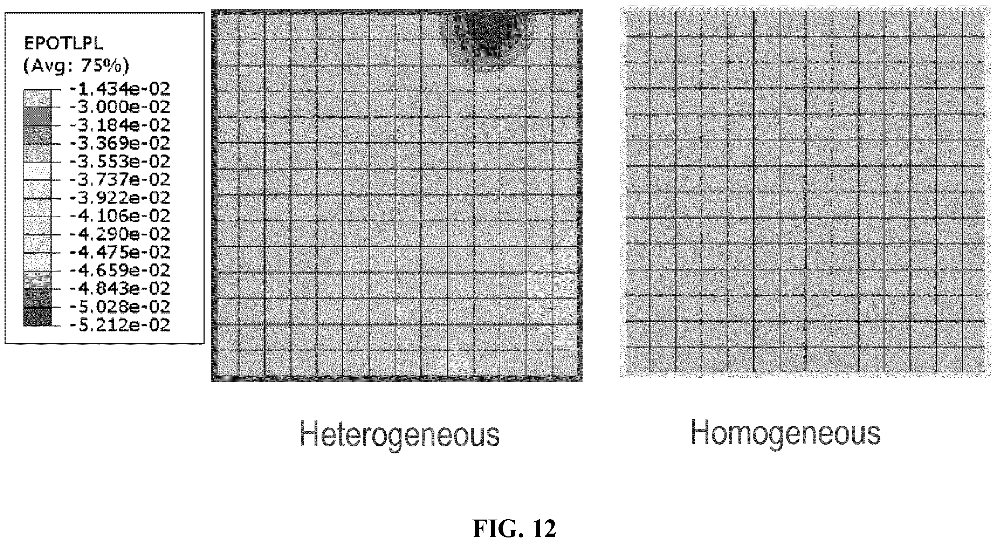

Since a higher plating potential correlates with less plating, this indicates that different regions of the anode near the separator will plate to different extents, whereas the homogeneous model would predict the same extent of plating in all regions of the cell at a given distance from the separator. FIG. 12 depicts contour plots of plating potential (EPOTLPL) at the face of the anode nearest the separator at the end of the charge phase.

Given that predicting the extent to which lithium plating occurs is crucial to predicting long term behavior in a battery, it is desirable to understand what regions of a cell tend to plate more than others. The workflow of the exemplary embodiment allows for this determination, which is unavailable with other battery simulation software.

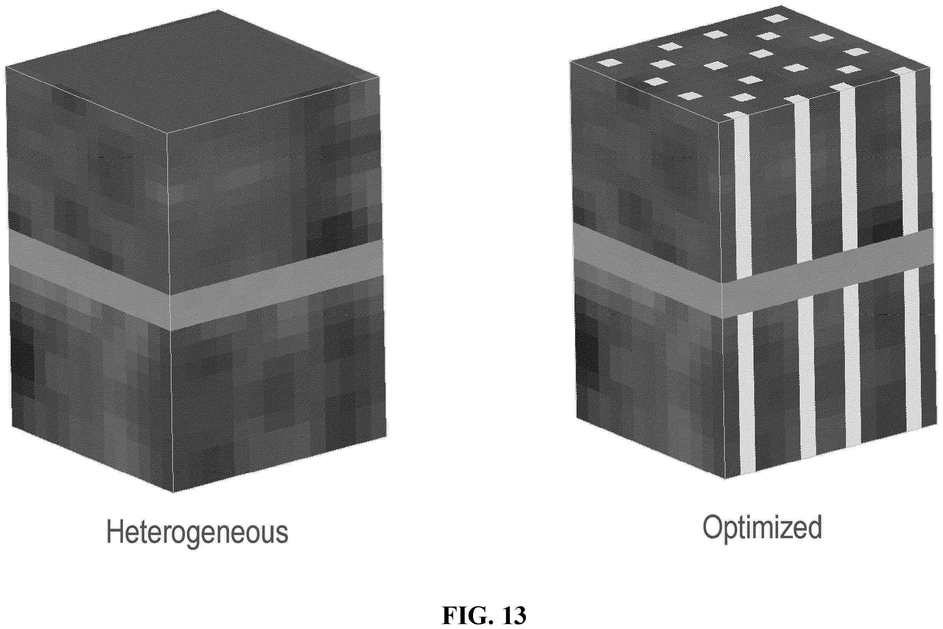

The subtle difference in performance between the heterogeneous and homogeneous models may be amplified by adjusting the porosity distribution. Experimental evidence (Yari et. al, 2020) suggests that certain distributions of particles, which would result in a certain distribution of porosity, promote better ionic transport within batteries. For example, introducing high porosity channels into anode and cathode materials via laser ablation or micro hole drilling could induce better transport properties (see Dunlap et. al, 2022). These lines of reasoning motivated the development of an optimized cell with an artificial porosity distribution, wherein high porosity "highways" span the thickness of the anode and cathode, but the average porosity of the anode, cathode, and separator is still equal to that of the respective region of the un-optimized heterogeneous cell previously considered, as shown in FIG. 13. Additionally, constitutive relationships and material properties are equivalent between models.

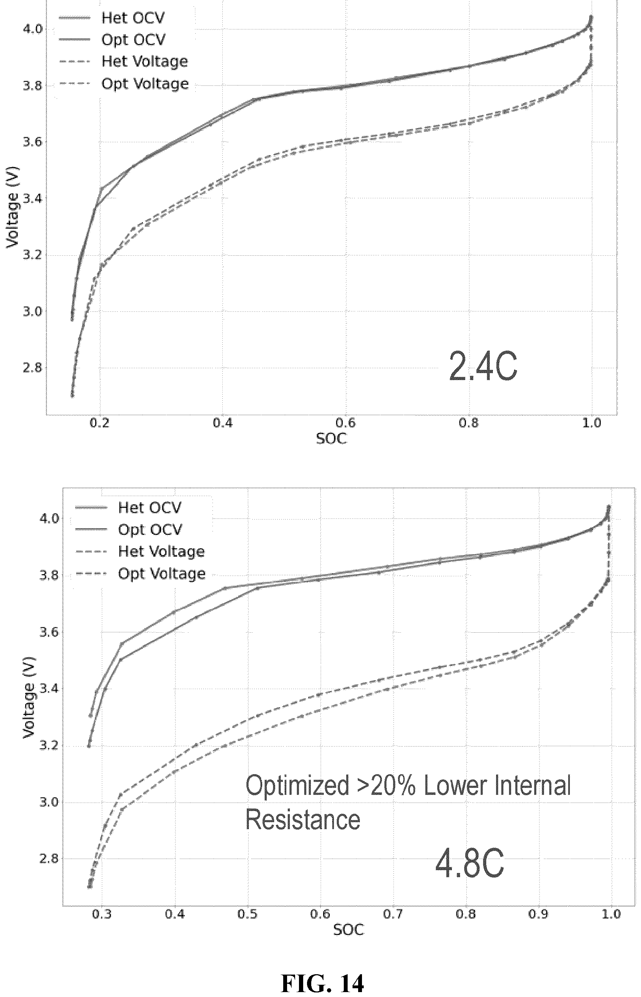

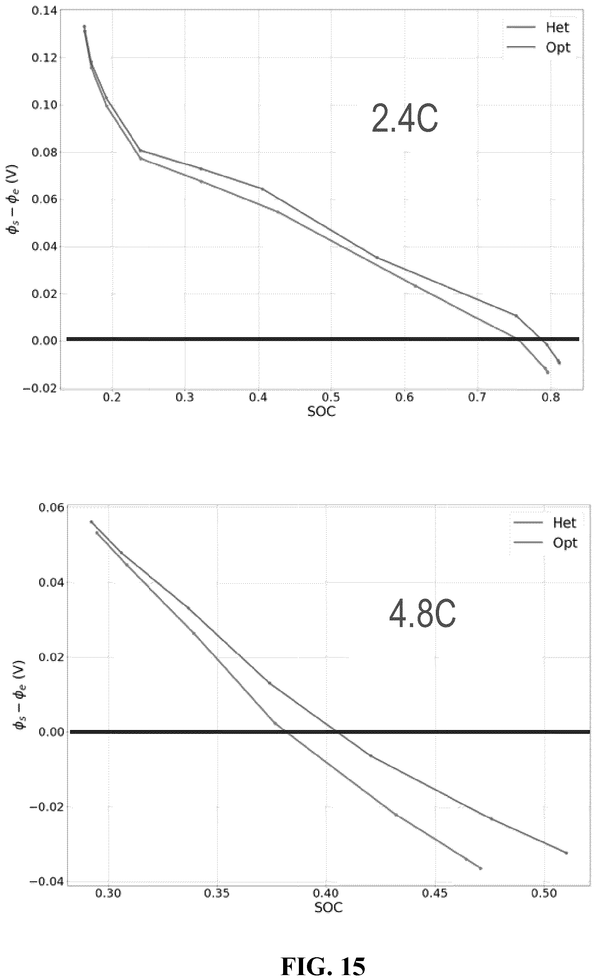

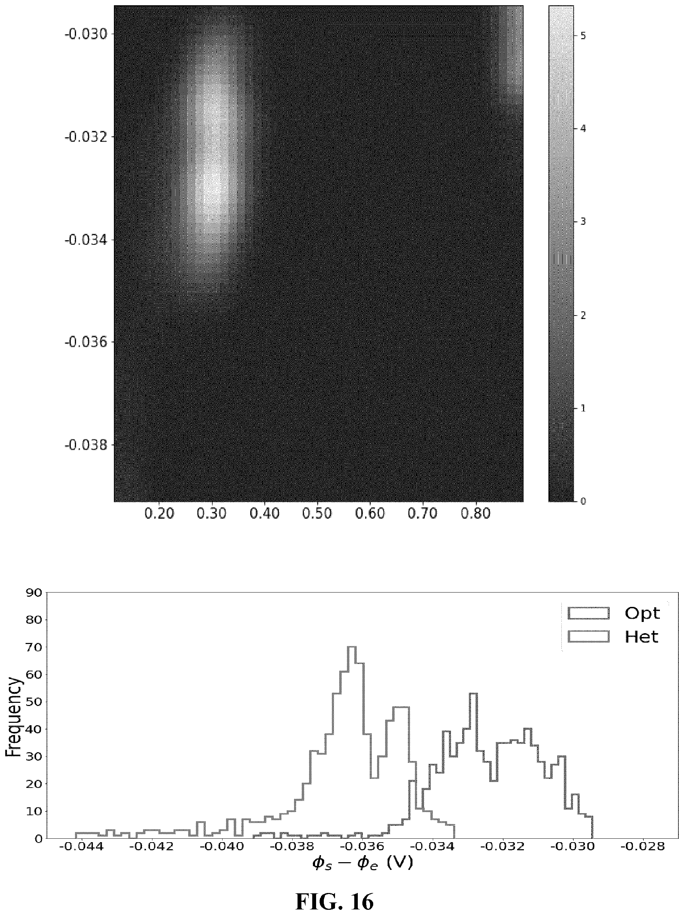

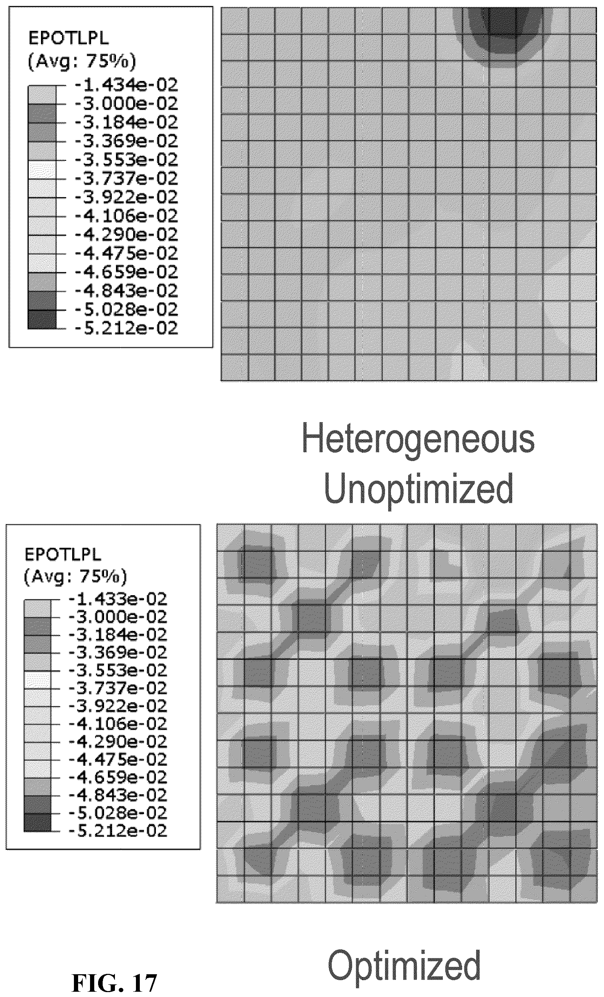

FIG. 14 depicts the voltage/OCV versus SOC curves at 2.4C and 4.8C for the heterogeneous and optimized batteries. With these cells, a noticeable difference in internal resistance between the cells is observed, even at a discharge current of 2.4C. As expected, this difference becomes more significant when the C rate is increased. Further, there is a drastic difference in the EPOTLPL between the optimized and heterogeneous cells, where the optimized cell is observed to plate at a state of charge that is eight percent higher than the state of charge at which the heterogeneous cell plates, as shown in FIG. 15. As such, the EPOTLPL in the optimized cell is typically higher than that of the heterogeneous cell at all points of the charge cycle. This fact is further emphasized by the distributions of EPOTLPL in the anode near the separator at the end of the charging phase shown in FIG. 16, where the distribution of the optimized cell is clearly skewed towards a higher EPOTLPL, meaning that less plating should occur during charging overall. As shown by the corresponding contour plots in FIG. 17, it is clear that the regions of high EPOTLPL which correspond to less plating are concentrated around the high porosity "highways," as would be expected from the correlation between porosity and EPOTLPL previously noted.

FIG. 18 is a flowchart of an exemplary embodiment of a method 130 for upscaling a microstructure (e.g., provided by DigitalROCK) to a continuum modeler (e.g., Abaqus) for subsequent modeling of, for example, a Li-ion battery. It should be noted that any process descriptions or blocks in flowcharts should be understood as representing modules, segments, portions of code, or steps that include one or more instructions for implementing specific logical functions in the process, and alternative implementations are included within the scope of the present invention in which functions may be executed out of order from that shown or discussed, including substantially concurrently or in reverse order, depending on the functionality involved, as would be understood by those reasonably skilled in the art of the present invention.

3D microstructures for battery components 110 (FIG. 1) are received, as shown by block 1810, for example, an anode, a cathode, a separator, and/or a CBD. For example, the 3D microstructures may be obtained via X-ray micro-tomography or procedurally. Bulk and/or pure material properties of the battery components 120 (FIG. 1) are received, as shown by block 1820, such as diffusivity, thermal conductivity, electric conductivity (solid), ionic conductivity (electrolyte), and elastic properties, among others.

Emergent properties from the battery component microstructures are calculated as a function of porosity, as shown by block 1830. For example, microstructure images may be uploaded into DigitalROCK as 8-bit RAW files. Here, DigitalROCK's porous media characterization capability may be used to calculate emergent properties as a function of porosity by first segmenting the microstructure into a number of regions (to have representative values at different porosities), and then calculating material properties for each sub region, for example via DigitalROCK's diffusion solver. The results from DigitalROCK may then be interpreted, for example via a python script. Here, Bruggeman coefficients may be calculated for each battery component sub region for effective ionic conductivity, electric and thermal conductivities, and diffusivity, as shown by block 1840. For example, built-in curve fitting functions in the ScyPy python script may be used to determine Bruggeman coefficients for effective ionic conductivity, electric conductivity, and diffusivity. For thermal conductivity, a generalized Bruggeman relationship of the form

$$\text{Keff} = A\varepsilon_s^a + \text{Ke (Eq. 4)}$$

is fit to the data with fitting parameters A and a, where the bulk thermal conductivity of the electrolyte is added to the right-hand side to ensure that a solid volume fraction of zero yields the thermal conductivity of the fluid. Given that Abaqus allows for the definition of orthotropic Bruggeman coefficients for the effective ionic conductivity and diffusivity in the electrolyte, these values alone are stored for the later generation of Abaqus input files. For effective electric and thermal conductivity, tables may be generated based on the calculated curves, which may be stored to be included in Abaqus input files, for example, under the ELECTRIC CONDUCTIVITY and CONDUCTIVITY keywords. Relationships between elastic properties and porosity, effective area and porosity, saturation and pore pressure, and additional saturation dependency for all constitutive relationships are also possible.

A heterogeneous mesoscale 3D battery model 140 (FIG. 1) is created, as shown by block 1850. For example, in DigitalROCK, one can also combine the anode, cathode, and separator materials into a single cell structure, as shown by block 1852. Each of the anode, cathode, and separator are separately partitioned into coarse voxels, as shown by block 1854. For example, starting with the single cell structure, the original 8-bit image may be coarsened to a 32-bit image in the sense that the 8-bit image may be broken down into larger voxels, where an average porosity is assigned to each voxel; hence, the 32-storage is needed to achieve accurate porosity reporting. The voxel sizes for each cell region (anode, cathode, separator) where a unique set of voxels may be defined with a uniform voxel size throughout. Note that x, y, and z dimensions of each voxel need not be the same, which may be accomplished when the images used from each material are cropped accordingly before they are combined. Preferably, a desired level of coarsening may be determined before setting up the model, so that the size of the model can be determined accordingly.

From the final, coarsened image, a raw file may be converted to a txt file, for example, via an executable that associates a porosity to each coarsened voxel via its position in the image. Two executables are then used for this purpose, to be used depending on the endianness of the images. The level of coarsening may be selected for a given particle size. An Abaqus input file may be created using the coarsened image and constitutive relationships. Preferably, the desired material properties (apart from those calculated above) are known to the user, for example, via experimental data or other sources.

The following describes an exemplary methodology for generating a singular battery cell with one dimensional loading. Input files are created for Abaqus based on provided microstructure 3D models 110 (FIG. 1) and bulk/pure material properties 120 (FIG. 1), for example, using a Python script as described above. The following provides details of one example of such a script, amongst other possible implementations. User inputs for the script may be text files containing parts of the input file that do not pertain to material properties calculated by DigitalROCK, omitting the geometry definitions. Additionally, the size of the anode and separator in pixels in the thickness direction may be provided, along with the (pixel) dimensions of the whole cell, a scale factor (for example, if a cell is 14 pixels in width but the physical size of the cell in that dimension is 140 micrometers, the scale factor would be 10-5), the number of cycles for the simulation (at least two cycles is recommended, as the simulation unrealistically starts at equilibrium for the first cycle), the C-rate to be used in simulation, a tag to determine if the cell is to be homogenous or heterogeneous, the txt file generated from the coarsened image, and the desired name of the input file to be generated. Variable x, y, and z dimensions may optionally be defined for the elements to be created in Abaqus (as a function of the scale factor); cubic elements with dimension equal to the scale factor are created by default.

The geometry generation and region assignments of this example rely heavily on the NumPy python package. First, nodal coordinates are generated based on NumPy arrays generated based on the number of voxels in each direction and the voxel size (default: scale factor). Then, a multi-dimensional grid with these coordinates is generated using NumPy's mesh grid feature, and these coordinates are stacked and transposed so that each row of an object called "node_coordinates" represents the x, y, and z coordinates of a node. Then, a unique, flattened index is assigned to each node using the mesh grid feature again on arrays of the dimensions of the cell, which is then raveled and converted to a dictionary, where the nodal coordinates are assigned to their corresponding node index. A grid is created corresponding to element indices, as opposed to node indices. From this grid, the element nodes are procedurally assigned to each element, depending on position in the grid (the node grid has one more row in each direction than the element grid, so assignments are determined accordingly). This grid is then transposed so that each row corresponds to a voxel and each column to one of the 8 element nodes. After this, element sets are defined via position in the thickness direction; hence why the user should define the anode and separator voxel thicknesses. This is done by first calculating the center of each element by averaging the y (thickness) coordinates in each element. Here, a new list containing only the y coordinates of each node is also determined so that the extreme faces of the cell can be determined later. Next, the anode, cathode, and separator node sets are determined via the voxel size, the pixel size of the anode and separator, and the center y coordinate of each element. That is, if the center of an element is below the extreme edge of the anode, the element is included in the anode set, if it is above that point but below the extreme edge of the separator it is included in the separator set, and otherwise it is included in the cathode set. Then, elements at either extreme end of the battery are included in the "ground" set (elements at the extreme end of the anode) or the "load" set (elements at the extreme end of the cathode). Finally, node sets are generated for each of these elements by adding the nodes from each of the elements to a list.

An array of zeros which becomes an array of porosities for each element is generated with dimensions equal to the number of voxels in the cell. The element indices are defined such that they correspond directly to the assignment of porosities in the txt file previously generated, so the porosities are assigned directly, based on their corresponding positions in the image. This array is then flattened to match the dimensions of the array containing columns of element node numbers. Although optional, the next step illustrates the power of this general approach to mesh generation: In running test cases, it may be desirable to translate the geometry down in the y direction by the thickness of the cell and rotate it 90 degrees. Since porosities are defined per element elements are defined via node numbers and node coordinates may be assigned to node numbers based on only their positions relative to one another in a grid, if the grid of nodal coordinates is rotated or translated all together then the element, element set, and porosity assignments are translated with it. Thus, all of the information may be effectively rearrange by translating all node y coordinates down and rotating the whole array by 90 degrees. At this point, the geometry is completely defined.

The remaining node set assignments left to be made relate to reference points set at the origin and a sensor node, which may be located at the extreme end of the cathode at the bottom corner opposite the origin in the x direction, which can be determined by looking in the node coordinates list for the desired coordinates, and then returning the index of the coordinates desired. Functions may be used to write element and node sets in the style of an Abaqus input file. These node and element sets are then written to an input file, along with information required be Abaqus pertaining to element type (QEC3D8 in this case), surface definition for the extreme faces, material definitions (it is assumed the materials are named as described by the Abaqus electrochemistry documentation), and orientation. The rest of the input file is printed per the provided text files from the user, and the material property curves given, along with porosities defined per the initial conditions.

After the geometry is procedurally generated, this information is printed to Abaqus input file, along with the material property definitions, porosity assignments for each element (where averages are assigned to the element sets if the anode, cathode, and separator are assumed to be homogenous), and step information with charge rates calculated as a function of the 1-C rate (included in the text files to be provided by the user) and with the number of steps depending on the number of cycles defined by the user (again, a single cycle at 1-C is to be provided by the user). Electrical conductivity is typically defined via a property table for bulk electric conductivity and the Bruggeman coefficients used in the electrolyte, and the simulation will not run unless this table is defined, so the bulk electric conductivity defined in this way must be set to zero when generating the input file. This makes the electric conductivity defined through the Bruggeman relationship zero, and then only then electric conductivity defined through the keyword ELECTRIC CONDUCTIVITY may be used. Given that both of this and the (thermal) CONDUCTIVITY keywords do not allow for dependency on solid volume fraction by default, a user subroutine USER FIELD is used with the GETVRM function to get the porosity, where the solid volume fraction can be computed as one minus the porosity and subsequently provided as a field variable.

With the creation of the input file, the upscaling methodology is complete, as the user is now able to run the model in Abaqus. Post processing may subsequently be done according to the specific needs of the user.

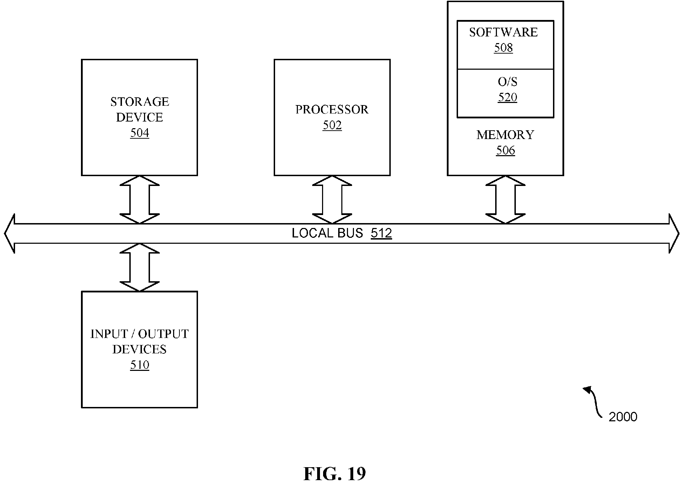

The present system for executing the functionality described in detail above may be a computer, an example of which is shown in the schematic diagram of FIG. 19. The system 2000 contains a processor 502, a storage device 504, a memory 506 having software 508 stored therein that defines the abovementioned functionality, input, and output (I/O) devices 510 (or peripherals), and a local bus, or local interface 512 allowing for communication within the system 2000. The local interface 512 can be, for example but not limited to, one or more buses or other wired or wireless connections, as is known in the art. The local interface 512 may have additional elements, which are omitted for simplicity, such as controllers, buffers (caches), drivers, repeaters, and receivers, to enable communications. Further, the local interface 512 may include address, control, and/or data connections to enable appropriate communications among the aforementioned components.

The processor 502 is a hardware device for executing software, particularly that stored in the memory 506. The processor 502 can be any custom made or commercially available single core or multi-core processor, a central processing unit (CPU), an auxiliary processor among several processors associated with the present system 2000, a semiconductor based microprocessor (in the form of a microchip or chip set), a macroprocessor, or generally any device for executing software instructions.

The memory 506 can include any one or combination of volatile memory elements (e.g., random access memory (RAM, such as DRAM, SRAM, SDRAM, *etc.*)) and nonvolatile memory elements (*e.g.,* ROM, hard drive, tape, CDROM, *etc.*). Moreover, the memory 506 may incorporate electronic, magnetic, optical, and/or other types of storage media. Note that the memory 506 can have a distributed architecture, where various components are situated remotely from one another, but can be accessed by the processor 502.

The software 508 defines functionality performed by the system 2000, in accordance with the present invention. The software 508 in the memory 506 may include one or more separate programs, each of which contains an ordered listing of executable instructions for implementing logical functions of the system 2000, as described below. The memory 506 may contain an operating system (O/S) 520. The operating system essentially controls the execution of programs within the system 2000 and provides scheduling, input-output control, file and data management, memory management, and communication control and related services.

The I/O devices 510 may include input devices, for example but not limited to, a keyboard, mouse, scanner, microphone, *etc.* Furthermore, the I/O devices 510 may also include output devices, for example but not limited to, a printer, display, *etc.* Finally, the I/O devices 510 may further include devices that communicate via both inputs and outputs, for instance but not limited to, a modulator/demodulator (modem; for accessing another device, system, or network), a radio frequency (RF) or other transceiver, a telephonic interface, a bridge, a router, or other device.

When the system 2000 is in operation, the processor 502 is configured to execute the software 508 stored within the memory 506, to communicate data to and from the memory 506, and to generally control operations of the system 2000 pursuant to the software 508, as explained above.

When the functionality of the system 2000 is in operation, the processor 502 is configured to execute the software 508 stored within the memory 506, to communicate data to and from the memory 506, and to generally control operations of the system 2000 pursuant to the software 508. The operating system 520 is read by the processor 502, perhaps buffered within the processor 502, and then executed.

When the system 2000 is implemented in software 508, it should be noted that instructions for implementing the system 2000 can be stored on any computer-readable medium for use by or in connection with any computer-related device, system, or method. Such a computer-readable medium may, in some embodiments, correspond to either or both the memory 506 or the storage device 504. In the context of this document, a computer-readable medium is an electronic, magnetic, optical, or other physical device or means that can contain or store a computer program for use by or in connection with a computer-related device, system, or method. Instructions for implementing the system can be embodied in any computer-readable medium for use by or in connection with the processor or other such instruction execution system, apparatus, or device. Although the processor 502 has been mentioned by way of example, such instruction execution system, apparatus, or device may, in some embodiments, be any computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the processor or other such instruction execution system, apparatus, or device.

Such a computer-readable medium can be, for example but not limited to, an electronic, magnetic, optical, electromagnetic, infrared, or semiconductor system, apparatus, device, or propagation medium. More specific examples (a nonexhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program can be electronically captured, via for instance optical scanning of the paper or other medium, then compiled, interpreted, or otherwise processed in a suitable manner if necessary, and then stored in a computer memory.

In an alternative embodiment, where the system 2000 is implemented in hardware, the system 2000 can be implemented with any or a combination of the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), etc.

Advantageously, the above described embodiments provides an efficient means of improving the accuracy of battery cell continuum model simulations without implementing an unfeasible number of degrees of freedom or drastically changing the well-known framework (Newman model). By moving away from generic microstructure model assumptions, like Bruggeman exponent 0.5, the embodiments allow correct capture of microstructural differences even between similar battery cells; for instance, electrodes with similar porosity can have different tortuosity values.

The embodiments provide a practical method for modeling aging and degradation mechanisms in a full cell driven by local extreme property values, for example, concentration, temperature, or plating potential. Performance captured by metrics such as internal resistance can be realized as a direct consequence of different microstructures, even those with the same average porosity in each material (anode, separator, cathode).

The embodiments further electrode microstructure optimization for both performance and aging, as shown in the detailed section for the optimized cell model that accurately captures the constructive nature of the heterogeneities. This result points to a clear potential for microstructure optimization for battery cell performance.

The optimized and heterogeneous cells are indistinguishable from one another in typical continuum battery modeling, as the anode, cathode, and separator materials have the same porosity between the models. Thus, they would both be modeled as the homogenous cell previously considered. By adjusting the size or location of these highways, or otherwise altering the porosity distribution further, it is possible that the performance of the battery cell may be improved further. Therefore, in addition to increased precision from more accurate constitutive relationships, the above described embodiments allow for battery microstructure optimization not possible in previous battery simulation techniques.

It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims and their equivalents.

**Claims**

1. A computer-implemented method for upscaling from a microstructure to generate a coarsened heterogeneous spatial distribution of porosity and a set of porosity dependent constitutive relationships, comprising the steps of:

   receiving a three dimensional (3D) microstructure model for each of a plurality of battery components, wherein the battery components comprise an anode, a cathode, and a separator;
   receiving bulk material properties and/or porosity for each of the battery components;
   calculating a coarsened porosity model with emergent properties from the battery component microstructures as a function of the porosity;
   for each battery component sub region, calculating Bruggeman coefficients from the group of effective ionic conductivity, electric and thermal conductivity, and ionic diffusivity; and
   creating a heterogeneous mesoscale 3D battery model, further comprising the steps of:

   combining the anode, cathode, and separator materials into a single cell structure; and
   separately partitioning each of the anode, cathode, and separator into coarse voxels to create a 3D model of porosity.

2. The computer-implemented method of claim 1, wherein the porosity of the received bulk material properties comprises a fine resolution model comprising a plurality of elements, wherein each element is characterized as either a solid having a porosity of substantially zero or a pore having porosity of 100%.

3. The computer-implemented method of claim 2, wherein calculating emergent properties from the battery component microstructures as a function of the porosity further comprises the steps of:

   computing an average porosity in sub regions of each material;
   assigning each element of the coarsened porosity model the average porosity for all elements within a region; and
   assigning the average porosities to individual elements of a finite element mesh.

4. The computer-implemented method of claim 1, wherein the voxels are uniquely defined with a uniform voxel size throughout for each of the anode, cathode, and separator.

5. The computer-implemented method of claim 1, further comprising the step of creating an Abaqus input file using the coarsened image and constitutive relationships.

6. The computer-implemented method of claim 1, further comprising the step of receiving the coarsened porosity model and associated constitutive relationships are received as input into a continuum electrochemical solver.

7. The computer-implemented method of claim 1, further comprising the step of:
   determining effective properties as a function of saturation.

8. The computer-implemented method of claim 1, further comprising the steps of:

   computing a capillary pressure vs saturation curve; and
   calculating a change in pore pressure with saturation changes.

9. The computer-implemented method of claim 6, further comprising the step of simulating a 3D Newman model (165), wherein the continuum electrochemical solver comprises a 3D Newman model simulator.

10. The computer-implemented method of claim 9, further comprising the step of computing performance and aging metrics (170) from degrees of freedom of the 3D Newman model (165).

11. The computer-implemented method of claim 10, further comprising the step of determining a point where lithium plating on a surface of the 3D Newman model (165) becomes thermodynamically favorable, optionally further comprising the step of optimizing microstructure of the anode and cathode materials of the 3D Newman model, optionally further comprising the step of introducing a plurality of high porosity channels into anode and cathode materials of the 3D Newman model.

12. The computer-implemented method of claim 1, further comprising the step of printing the heterogeneous mesoscale 3D battery model to a file.

13. The computer-implemented method of claim 12, further comprising the step of providing the heterogeneous mesoscale 3D battery model file to a continuum modeler.

14. A computer program comprising instructions which, when executed by a computer system, cause the computer system to perform the method according to any one of claims 1 to 13.

15. A computer system comprising a processor coupled to a memory, the memory having recorded thereon the computer program of claim 14.

FIG. 1

Lower Tortuosity      Higher Tortuosity

**FIG. 2**

Full Anode
Scan

Compute $D_e$, $K_e$, $\varepsilon_e$ for
material subvolumes

AnodeCBD Effective Diffusivity

Fit
Bruggeman
Coefficients

Porosity/Electrolyte Volume Fraction

**FIG. 3**

**effective ion diffusivity (electrolyte)**

$$\frac{D_e^{eff}(\phi)}{D_e}$$

x, z
y

$\phi$

**effective ionic conductivity (electrolyte)**

$$\frac{K_e^{eff}(\phi)}{K_e}$$

x, z
y

$\phi$

**effective electric conductivity (solid)**

$$\frac{K_s^{eff}(\phi)}{K_s}$$

y
x, z

$\phi$

**effective thermal conductivity (solid + electrolyte)**

$$\frac{\kappa^{eff}(\phi)}{\kappa}$$

y
x, z

$\phi$

**FIG. 4**

EP 4 546 203 A1

# Base case (common assumption)

| Region | Electrolyte ($\alpha_x$, $\alpha_y$, $\alpha_z$) | Solid ($\alpha_x$, $\alpha_y$, $\alpha_z$) |
|---|---|---|
| Anode | 0.5, 0.5, 0.5 | 0.5, 0.5, 0.5 |
| Cathode | 0.5, 0.5, 0.5 | 0.5, 0.5, 0.5 |
| Separator | 0.5, 0.5, 0.5 | N/A |

# Homogeneous case (from real microstructure)

+

| Region | Electrolyte ($\alpha_x$, $\alpha_y$, $\alpha_z$) | Solid ($\alpha_x$, $\alpha_y$, $\alpha_z$) |
|---|---|---|
| Anode | 0.77, 1.05, 0.74 | 0.67, 1.17, 0.62 |
| Cathode | 0.92, 1.11, 0.94 | 0.57, 0.62, 0.54 |
| Separator | 0.59, 4.06, 0.52 | N/A |

**FIG. 5**

EP 4 546 203 A1

**FIG. 6**

Original Scan

Heterogeneous

Homogeneous

cathode    separator    anode

Coarser

Coarser

150 x 10$^6$ voxels

2700 Voxels

EP 4 546 203 A1

**FIG. 7**

FIG. 8

**FIG. 9**

3C

6C

FIG. 10

FIG. 11

Homogeneous

Heterogeneous

FIG. 12

EPOTLPL
(Avg: 75%)

-1.434e-02
-3.000e-02
-3.184e-02
-3.369e-02
-3.553e-02
-3.737e-02
-3.922e-02
-4.106e-02
-4.290e-02
-4.475e-02
-4.659e-02
-4.843e-02
-5.028e-02
-5.212e-02

Heterogeneous

Optimized

**FIG. 13**

**FIG. 14**

**FIG. 15**

FIG. 16

EPOTLPL
(Avg: 75%)
-1.434e-02
-3.000e-02
-3.184e-02
-3.369e-02
-3.553e-02
-3.737e-02
-3.922e-02
-4.106e-02
-4.290e-02
-4.475e-02
-4.659e-02
-4.843e-02
-5.028e-02
-5.212e-02

## Heterogeneous
## Unoptimized

EPOTLPL
(Avg: 75%)
-1.433e-02
-3.000e-02
-3.184e-02
-3.369e-02
-3.553e-02
-3.737e-02
-3.922e-02
-4.106e-02
-4.290e-02
-4.475e-02
-4.659e-02
-4.843e-02
-5.028e-02
-5.212e-02

**FIG. 17**

## Optimized

Receive microstructures for battery components <u>1810</u>

Receive bulk properties for the battery components, <u>1820</u>

Calculate emergent properties from the battery component microstuctures as a function of porosity <u>1830</u>

For each battery component sub region, calculate Bruggeman coefficients for effective ionic conductivity, electric conductivity, and diffusivity
<u>1840</u>

Create a heterogeneous mesoscale 3D battery model
<u>1850</u>

Combine the anode, cathode, and separator materials into a single cell structure
<u>1852</u>

partition each of the anode, cathode, and separator into coarse voxels
<u>1854</u>

130

**FIG. 18**

STORAGE
DEVICE
504

PROCESSOR
502

SOFTWARE
508

O/S
520

MEMORY
506

LOCAL BUS 512

INPUT / OUTPUT
DEVICES
510

2000

**FIG. 19**

Europäisches
Patentamt

European
Patent Office

Office européen
des brevets

# EUROPEAN SEARCH REPORT

**Application Number**

EP 24 20 8442

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHUO MINGZHAO ET AL: "Tensorial effective transport properties of Li-ion battery separators elucidated by computational multiscale modeling", ELECTROCHIMICA ACTA, ELSEVIER, AMSTERDAM, NL, vol. 393, 12 August 2021 (2021-08-12), XP086758056, ISSN: 0013-4686, DOI: 10.1016/J.ELECTACTA.2021.139045 [retrieved on 2021-08-12] * abstract * * sections 2.1, 2.2, 2.3, 2.4, 2.5 eq. 1, 6, 11-15, 18; figures 1, 2, 4; tables 1, 2 * | 1-15 | INV. G06F30/23 H01M10/0525 ADD. G06F111/10 G06F119/04 H01M4/02 |
| X | LANDSTORFER M ET AL: "A Modeling Framework for Efficient Reduced Order Simulations of Parametrized Lithium-Ion Battery Cells", ARXIV.ORG, CORNELL UNIVERSITY LIBRARY, 201 OLIN LIBRARY CORNELL UNIVERSITY ITHACA, NY 14853, 12 October 2021 (2021-10-12), XP091075038, * abstract * * section 2.2, section 3; figures 1, 2, 4 * | 1-15 | |

-----

-----

-/--

**TECHNICAL FIELDS SEARCHED (IPC)**

G06F
H01M

The present search report has been drawn up for all claims

| Place of search | Date of completion of the search | Examiner |
|---|---|---|
| Munich | 12 February 2025 | Brandiska, Pavlina |

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AWARKE ALI ET AL: "Quantifying the effects of strains on the conductivity and porosity of LiFePO4 based Li-ion composite cathodes using a multi-scale approach", COMPUTATIONAL MATERIALS SCIENCE, ELSEVIER, AMSTERDAM, NL, vol. 50, no. 3, 13 November 2010 (2010-11-13), pages 871-879, XP087109837, ISSN: 0927-0256, DOI: 10.1016/J.COMMATSCI.2010.10.024 [retrieved on 2010-11-13] * abstract * * section 3, 4, 7; figures 1, 6; table 1 * ----- | 1-15 | |

TECHNICAL FIELDS
SEARCHED        (IPC)

The present search report has been drawn up for all claims

| Place of search | Date of completion of the search | Examiner |
|---|---|---|
| Munich | 12 February 2025 | Brandiska, Pavlina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63592325

**Non-patent literature cited in the description**

- **ALLEN, J.M.** ; **CHANG, J.** ; **USSEGLIO-VIRETTA, F.L.E. et al.** A Segregated Approach for Modeling the Electrochemistry in the 3-D Microstructure of Li-Ion Batteries and Its Acceleration Using Block Preconditioners. *J Sci Comput*, 2021, vol. 86, 42
- **MADELEINE ECKER**. Parameterization of a Physico-Chemical Model of a Lithium-Ion Battery: I. Determination of Parameters. *J. Electrochem. Soc.*, 2015, vol. 162, A1836
- **MADELEINE ECKER**. Parameterization of a Physico-Chemical Model of a Lithium-Ion Battery: II. Model Validation. *J. Electrochem. Soc.*, 2015, vol. 162, A1849
- **ANDREW COLCLASURE**. Quantifying Heterogeneities/Degradation During Fast Charge. *Presentation, DOE Vehicle Technologies Profram 2020 Annual Merit Review and Peer Evaluation Meeting, June 1-4, 2020*
- **MATTHEW KEYSER** ; **KANDLER SMITH** ; **HAKIM IDDIR**. Understanding Impact of Local Heterogeneities During Fast Charge. *Presentation, U.S. Department of Energy Vehicle Technologies Office Annual Merit Review, Arlington VA, June 10-13, 2019*